# EUROPEAN PATENT SPECIFICATION

(11) **EP 0 380 542 B1**
(45) Date of publication and mention of the grant of the patent: **24.04.1996**
(21) Application number: 88908490.1
(22) Date of filing: 11.08.1988
(51) Int. Cl.: A61K 51/10, A61K 39/395

(54) **METHOD FOR TREATING MALIGNANCY AND AUTOIMMUNE DISORDERS IN HUMANS**
VERFAHREN ZUR BEHANDLUNG DER BÖSARTIGEN UND AUTOIMMUNEN KRANKHEITEN BEIM MENSCHEN
PROCEDE DE TRAITEMENT DE MALIGNITE ET DE TROUBLES D'AUTO-IMMUNITE CHEZ LES ETRES HUMAINS

(30) Priority: 17.08.1987 US 85707
(43) Date of publication of application: 08.08.1990
(73) Proprietor: THE UNITED STATES OF AMERICA as represented by the Secretary United States Department of Commerce, Springfield, Virginia 22161 (US)
(72) Inventor: WALDMANN, Thomas, A., Silver Spring, MD 20902 (US)
(74) Representative: Lord, Hilton David
(86) International application number: US8802731
(87) International publication number: WO8901340

(56) References cited:
- EP-A- 0 217 922
- EP-A- 0 240 344
- EP-A- 0 241 811
- EP-A- 0 296 082
- DE-A- 2 011 612
- SU-A- 0 438 419
- CHEMICAL ABSTRACTS, vol. 107, 1987, Columbus, OH (US); p. 654, no. 23714c
- JOURNAL OF EXPERIMENTAL MEDICINE, vol. 162, The Rockefeller University Press; R.L. KIRKMAN et al., pp. 358-362
- THE LANCET, vol. 1, 1987; J.P. SOULILLOU et al., pp. 1339-1342
- PROCEEDINGS OF THE NATL. ACADEMY OF SCIENCES USA, vol. 83, January 1986, Washington, DC (US); R.W. KOZAK et al., pp. 474, 477-478
- JOURNAL OF CLINICAL INVESTIGATION, vol. 74, September 1984; D.J.P. FITZGERALD et al., p. 967
- BLOOD, vol. 65, no. 6, June 1985; M. KRONKE et al., p. 1417

## Description

### BACKGROUND OF THE INVENTION

### Technical Field:

The present invention is related to a method for treating malignancy and autoimmune disorders and for preventing allograft rejection. More particularly, the present invention is directed to treating any human condition or disorder related to the expression of Tac antigen or involving abnormal IL-2- receptor expression, by reacting Tac antigen or IL-2 receptor expressing cells with anti-Tac monoclonal antibody or a preparation thereof.

### State of the Art:

The normal resting cells of the body, including T cells, do not express IL-2 receptors and thus do not react with a monoclonal antibody anti-Tac that recognizes the human IL-2 receptor. However, in certain conditions, such as in leukemic T cells of patients infected with human T-cell lymphotrophic virus I (HTLV-I-associated Adult T Cell Leukemia), large numbers of IL-2 receptors are constitutively expressed. The Tac antigen is also expressed in other malignant conditions including the malignant B lymphocytes of hairy cell leukemia, follicular lymphoma and the Reed-Sternberg cells of Hodgkin's disease. Furthermore, activated T cells expressing the Tac antigen also appear to play a pathogenic role in certain forms of autoimmune disorders, such as type I diabetes and a subset of patients with aplastic anemia. In addition, when cells responding to foreign histocompatibility antigens become activated, they express the Tac antigen and participate in allograft rejection such as in patients receiving vascularized organ allografts and in graft-versus-host disease in patients receiving marrow allografts. Thus, there are a number of clinical circumstances where the expression of Tac-antigen is involved. Clearly, therefore, the elimination of Tac-positive cells using the anti-Tac monoclonal antibodies would be of value in treating or controlling such pathological states.

Chemical Abstracts 107 (1987), 23714c, teaches that radioactive medicinal compositions have a prolonged active life if they are prepared by mixing an yttrium chloride colloidal solution containing yttrium-90 and gelatinol with a stabilising solution and adding the resulting mixture to a solution of potassium oleate and disodium hydrogen phosphate gelatinol.

In Proceedings of the National Academy of Sciences, 83, (1986), 474-478, there is taught the use of anti-Tac monoclonal antibodies conjugated with an α-emitting bismuth isotope for the treatment of adult T-cell leukaemia. However, suitable bismuth isotopes only have a half life of about one hour.

### SUMMARY OF THE INVENTION

It is, therefore, an object of the present invention to provide a method of eliminating disease-associated Tac-positive cells.

It is a further object of the present invention to provide a method of treating adult T-cell leukemia or T-cell-mediated autoimmune disorders.

It is another object of the present invention to provide a method of treating B-cell malignancy.

It is yet another object of the present invention to provide a method of controlling allograft rejection reactions.

Other objects and advantages of the present invention will become apparent from the Detailed Description of the Invention.

### BRIEF DESCRIPTION OF THE DRAWINGS

These and other objects, features and many of the attendant advantages of the invention will be better understood upon a reading of the following detailed description when considered in connection with the accompanying drawings wherein:

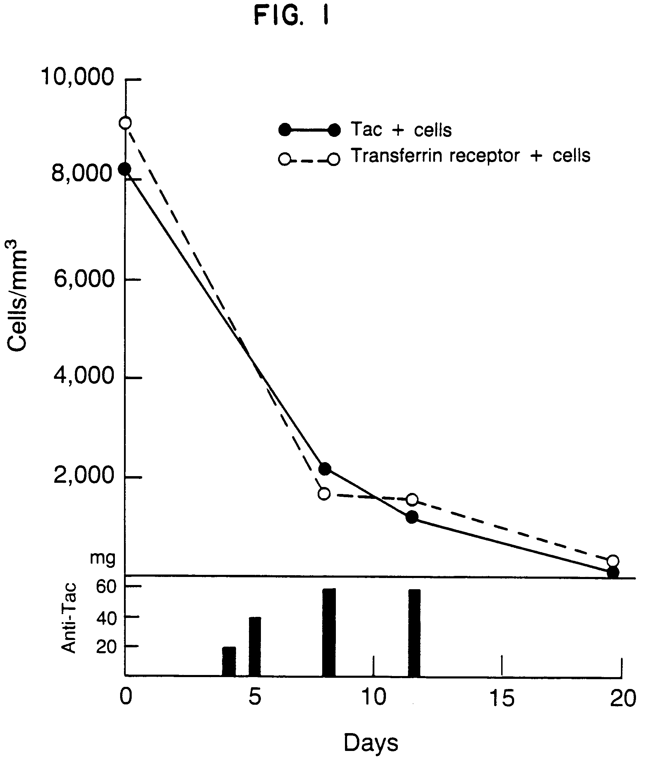

Fig. 1 shows the results of anti-Tac therapy of patient with Tac-positive ATL. The patient was treated with four infusions (20, 40, 50, and 50 mg) of anti-Tac monoclonal antibody over a 12 day period (indicated by solid bars). After the anti-Tac therapy, the number of circulating T cells bearing the Tac antigen declined from 8000 to less than 100/mm³. There was a parallel decline of cells expressing another tumor-associated marker of the transferrin receptor from over 9000 before therapy to less than 100/mm³;

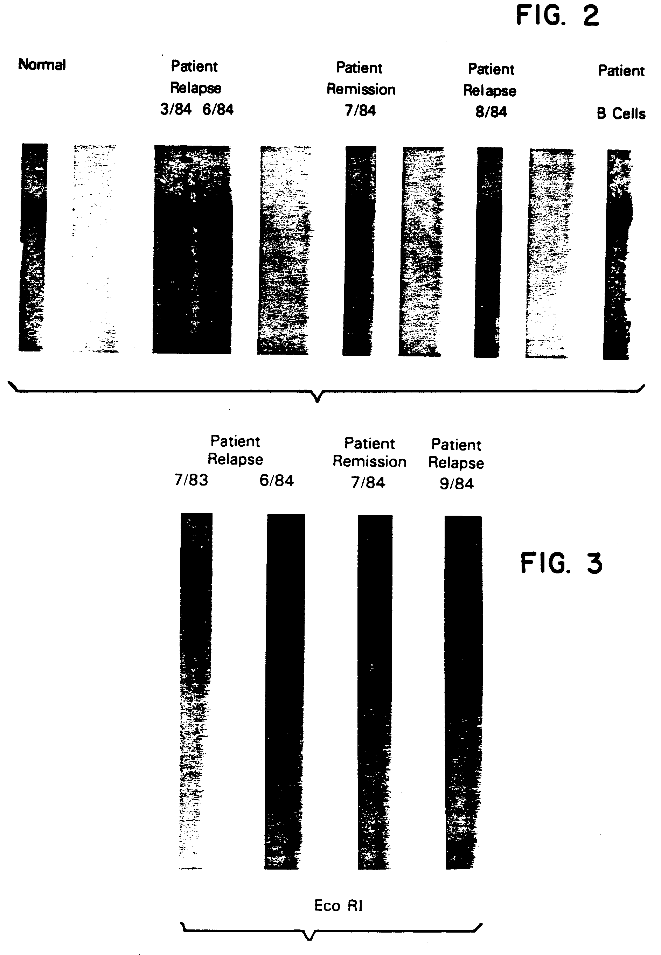

Fig. 2 shows the effect of anti-Tac therapy on CT β chain gene arrangement in a patient with ATL. The remission of the T-cell leukemia in this patient after anti-Tac therapy was confirmed using molecular genetic analysis of the arrangement of the genes encoding the β chain of the antigen-specific T-cell receptor. Southern analysis of the arrangement of the T-cell receptor β chain was performed on BamHI digests of DNA from the peripheral blood mononuclear cells of the patient by using a radiolabeled probe to the constant region of the T β chain. The constant T β genes are universally present on a 24-kb BamHI fragment in germline tissues of normal individuals and in a B-cell line from the patient. However, before therapy there was an additional 22-kb BamHI band hybridizing with the constant T β probe when digests of the patient's circulating T cells were examined, a hallmak of a clonal expansion of T lymphocytes. This band reflecting the clonally rearranged T-cell receptor gene was not demonstrable on specimens obtained after anti-Tac therapy when the patient was in remission. Six months after the initial remission the leukemia recurred with reappearance of leukemic cells identified by a molecular genetic analysis. A second course of infusions of anti-Tac was followed by a virtual disappearance of the skin lesions and the circulating leukemic cells (data not shown); and
Fig. 3 shows the effect of anti-Tac therapy on leukemic mononuclear cells with integrated HTLV-I. HTLV-I is clonally integrated into the cells of patients with HTLV-I-associated ATL. Such integrated HTLV-I can be identified by Southern analysis using a radiolabeled HTLV-I probe. In the case shown, there are two lines on the Southern gel indicating the integration of two HTLV-I viruses per cell. After anti-Tac therapy, the circulating cells of this patient did not contain integrated HTLV-I as shown by the clear Southern gel radioautograph. After relaps, integrated HTLV-I could again be demonstrated in the circulation T cells.

### DETAILED DESCRIPTION OF THE INVENTION

The above and various other objects and advantages of the present invention are achieved in accordance with claims 1 and 2. Unless defined otherwise, all technical and scientific terms used herein have the same meaning as commonly understood by one of ordinary skill in the art to which this invention belongs. Although any methods and materials similar or equivalent to those described herein can be used in the practice or testing of the present invention, the preferred methods and materials are now described.

Prior to the present studies, little was known about the inducible IL-2 receptor, and no antibodies to IL-2 receptor had been made. Using hybridoma technology, an IgG2a mouse monoclonal antibody called anti-Tac was prepared. This anti-Tac antibody reacted with activated but not resting T cell (Uchiyama et al, J. Immunol. 126:1393-1397, 1981; Uchiyama et al, J. Immunol. 126:1390-1403, 1981). Furthermore, this antibody identified the IL-2 receptor and blocked IL-2 binding to its receptor (Leonard et al, Nature 300:267-269, 1981). The structure, function and expression of the IL-2 receptors on normal and malignant lymphocytes has been reviewed by Waldmann (Science, 232:727-732, 1986).

Based on the known unique properties of anti-Tac antibodies, an approach to immunotherapy was developed to eliminate leukemic cells and activated T cells in autoimmune disorders and in organ allograft protocols. The therapeutic studies were extended by coupling toxins to anti-Tac and showing that they killed tumor cells at doses that did not affect normal cells. Furthermore, anti-Tac was coupled to the alpha-emitting radionuclide such as bismuth 212 (²¹²Bi) or, in accordance with the invention, the β-emitting radionuclide yttrium-90 by the use of a bifunctional chelate. This agent was also shown to be an effective and specific immunocytotoxic agent for the elimination of IL-2 receptor-positive cells. The details of the procedure for the use of anti-Tac in the therapy of patients with adult T-cell leukemia and in organ allograft protocols are described below.

### A. Comparitive Treatment of ATL with Unmodified Anti-Tac

Patients with adult T-cell leukemia (ATL) are treated by intravenous infusions of unmodified anti-Tac. ATL is an aggressive leukemia of polymorphic mature T cells with a propensity to infiltrate the skin. This leukemia is frequently associated with hypercalcemia and pulmonary involvement. The leukemic cells always contain the C-type retrovirus Human T-Cell Lymphotrophic Virus I (HTLV-I). There is no curative threapy for patients with ATL, and such patients have a mean survival time of only about 20 weeks. In contrast to normal cells, the malignant cells of patients with ATL display the cell surface receptor for interleukin-2 identified by the anti-Tac monoclonal antibody.

The anti-Tac murine-derived monoclonal antibody used for these therapeutic studies has been produced by fusing NS-1 cells with spleen cells of mice immunized with a cell line derived from an ATL patient. Large quantities of the monoclonal antibody are produced by inoculating hybrid cells into the peritoneum of BALB/c mice and purifying this IgG2a κ antibody from the resulting ascites fluid by DEAE chromatography with elution by 0.1 Tris buffer as the eluting agent. The material is dialyzed against saline, centrifuged, filtered, precipitated with 20% sodium sulfate, and then diluted in saline at pH 7.4 to a concentration of about 2 mg/ml. Each lot of the product is shown to be pure by assays that include immunoelectrophoresis, diffusion in agar plates using antisera to IgG2a, IgG1, IgM, and transferrin, as well as polyvalent antibodies to major mouse proteins. Furthermore, the lots are shown to be homogenous by HPLC. The monoclonal preparations are sterilized by passge through a 0.22 millipore filter and are shown to be nonpyrogenic and sterile. Patients with Tac-expressing ATL receive anti-Tac antibody by intravenous administration of a dose in 100 cc of normal saline with 5% human albumin over a 2-hour period. Patients receive anti-Tac at a higher dosage each week for 2 weeks. During the first week, they receive two doses of 20 mg per patient and, in the second week, two doses of 50 mg per patient. Patients undergoing partial or complete remission or those with leukemic cells with persistent Tac receptors unblocked by the anti-Tac monoclonal antibodies may receive additional biweekly doses of 50 mg of anti-Tac. Unblocked IL-2 receptors can be identified with flow cytometry using fluorochrome-labeled anti-Tac monoclonal antibodies using conventional procedures.

Ten patients with ATL have been treated with intravenously administered anti-Tac monoclonal antibody according to the above protocol. None of the patients suffered any untoward reactions or produced antibodies to mouse immunoglobulin or to the idiotype of the anti-Tac monoclonal. There was no reduction in the number of any of the normally formed elements of the blood. Three of the patients had a reduction in the number of circulating leukemic cells or a complete remission after anti-Tac therapy. In one of these patients, therapy was followed by a 5-month remission as assessed by routine hematological tests, immunofluorescence analysis of circulating T cells (Figure 1), molecular genetic analysis of the arangement of the genes encoding the chain of the T-cell receptor (Figure 2), as well as the genes of the retrovirus HTLV-1 (Figure 3). After the 5-month remission, the patient's disease relapsed, but a new course of anti-Tac therapy was followed by a virtual disappearance of the skin lesions and an over 80% reduction in the number of circulating leukemic cells.

### B. Comparitive Treatment of ATL with Anti-Tac Conjugated with Cytotoxic Agents

### 1. Anti-Tac Antibody Coupled to Ricin A Chain

Using conventional procedures, purified anti-Tac monoclonal antibody is conjugated to purified or recombinant ricin A chain using a thiol-containing crosslinker, N-succinimidyl-3-(2-pyridyldithio)propionate (Kronke et al Blood 65:1416-1421, 1985). The resulting conjugates are separated from the majority of free ricin A chains by Sephacryl S-200 gel filtration. Conjugates are adjusted to 1 mg/ml with reduced and alkylated human IgG and stored at -20°C. The addition of carrier protein assures stability of the conjugates, and the alkylation prevents disulfide toxin exchange between specific antibody and carrier protein. The addition of anti-Tac antibody coupled to the A chain of the toxin (ricin) effectively inhibited protein synthesis and led to cell death of an HTLV-I-associated, Tac-positive ATL cell line, HUT102-B2. In contrast, conjugates of ricin A with a control monoclonal of the same isotype did not inhibit protein synthesis when used in the same concentration. The inhibitory action of anti-Tac conjugated with ricin A could be abolished by the addition of excess unlabeled anti-Tac or IL-2.

### 2. Anti-Tac Coupled to Pseudomonas Toxin

The immunotoxin Pseudomonas exotoxin anti-tac is made from purified pyrogen-free anti-Tac and purified Pseudomonas exotoxin (PE) according to published methods (Fitsgerald et al Proc. Natl. Acad. Sci. USA 80:4134-4138, 1983). Two mg (30 nM) of PE in KPO₄ 0.1 M, EGTA 1 mM, pH 8.0, is incubated with 500 nM of NAD and 5000 nM of 2-iminothiolane-HCl for 1 hour at 37°C. NAD is added to protect the enzyme-active site of the toxin. This derivatized PE preparation is separated on HPLC from a small amount of aggregated toxin by the other reactants. Dithio-bis(2-nitrobenzoic acid) (DTNB) is added to the derivatized PE to a final concentration of about 1mM. The addition of DTNB and its reaction with free sulfhydryl groups serves to activate the toxin for future disulfide exchange with antibody.

The antibody (5-8 mg) in KPO₄ 0.1 M, EGTA 1 mM, pH 8.0, is incubated with 120 nMol of 2-iminothiolane-HCL for 1 hour at 37°C. At the end of the incubation period, the antibody is separated from iminothiolane by gel filtration on a G-25 column. An aliquot of the derivatized antibody is reacted with DTNB to determine the number of new sulfhydryl groups introduced. The remainder is mixed with the activated PE. Activated PE is reacted with derivatized anti-Tac antibody. The reaction is followed by measuring the release of TNB (thionitro-benzoic acid - nitrophenol) at OD₄₁₂. The antibody-SH releases routinely half of the TNB from the activated PE molecules. The balance is released by adding excess cysteine. The reaction mixture is separated by HPLC. The PE-antibody-(cys)₂ has the most activity and is used for patient therapy. The PE-anti-Tac is stored at -20°C in 0.15 M NaCl, 10 mM KPO₄, 1 mM EGTA, pH 7.2.

Patients with ATL receive PE-anti-Tac antibody by intravenous administration in 100 cc of normal saline with 1% albumin over 2 hours. Each patient received about 200 g of PE-anti-Tac twice during the first week and 2 mg twice a week during the second week. Therapy is stopped if the patient manifests grade III hepatic toxicity, that is, a bilirubin over 3.0 mg/ml or an SGOT or alkaline phosphatase 3-5 times the base line.

Four patients have been treated with PE-anti-Tac according to this protocol. One of the four patients manifested hepatic dysfunction, including abdominal pain and a transient disorder of the liver function tests. One of the patients had a response to the PE-anti-Tac therapy manifested by an over 50% decline in the number of circulating leukemic cells.

It is noted that other cytotoxic conjugates of anti-Tac can be similarly prepared and used. The examples provided herein being only exemplary.

### C. Therapy of ATL with Anti-Tac Conjugated with Radionuclides

Anti-Tac has been successfully conjucated to the α-particle-emitting radionuclide bismuth-212 and, in accordance with the invention, to the β-emitting yttrium-90 by use of bifunctional ligands, such as isobutylcarboxycarbonic anhydride of diethylenetriamine-pentaacetic acid (DTPA). The physical properties of ²¹²Bi are appropriate for radioimmunotherapy in that it has a short half-life, deposits its high energy over a short distance, and can be obtained in large quantities from a radium generator. The labeling protocols have been described by Gansow et al (Am. Chem. Soc. Symp. Ser. 241:215-227). DTPA is linked to anti-Tac with ¹⁴C-labeled DTPA used to identify chelate-antibody ratio. DPA (0.2 mM)was dissolved in 2 ml of H₂O by addition of triethylamine (1.38 mM) and lyophilized. The solid formed is taken up in 1 ml of acetonitrile at 4°C and treated with isobutylchloroformate (0.27 mM) for about 30 minutes, centrifuged, and a 20-µl aliquot of isobutylcarboxycarbonic anhydride solution is reacted with anti-Tac at 4°C for about 1.5 hours. Sequential dialyses in metal-free buffer are used to purify the protein. A comparable procedure is used to couple anti-Tac to the β-emitting radionuclide yttrium-90. Conjugates with other α or β emitting nuclides are similarly prepared and used. The examples provided herein being only exemplary.

Activity levels of 0.5 µCi or the equivalent of 12 rad/ml of α irradiation targeted by ²¹²Bi-anti-Tac eliminated more than 98% of the proliferative capacity of the HUT102-B2 cells with only minimal effect on IL-2 receptor-negative lines. This specific cytotoxicity was blocked by excess unlabeled anti-Tac but not by human IgG. Thus, ²¹²Bi-anti-Tac is an effective and specific immunocytotoxic agent for the elimination of IL-2 receptor-positive ATL cells.

### D. Comparitive Protocol for Treatment of Autoimmune Disorders

Patients with certain forms of autoimmune disease, including subsets of patients with the disease aplastic anemia, have increased number of circulating and marrow Tac-positive T cells. In this group of patients, the Tac-positive but not the Tac-negative T cells inhibit hematopoiesis when cocultured with normal bone marrow cells. Patients with elevated number of Tac-positive T cells and associated aplastic anemia receive unmodified anti-Tac monoclonal antibody in 100 ml normal saline with 5% alumin by intravenous administration over a 2 hour period. Patients are treated with 20 mg of anti-Tac three times over a 7 to 10 day period. This course may be modified and repeated if Tac positive cells remain elevated.

An alternative therapeutic approach with anti-Tac is the use of Pseudomonas exotoxin anti-Tac according to protocols described above. Patients receive about 200 ug of PE-anti-Tac twice a week during the first week of treatment and at doses of about 2 mg twice a week during the second week.

### E. Comparitive Protocol for Treatment to Prevent Allograft Rejection

After renal or cardiac allografts and during graft-versus-host diesase, certain host T lymphocytes recognize the foreign histocompatibility antigens expressed on the donor organs and thus become activated and express the Tac antigen. Such Tac-expressing activated T cells participate in the rejection of the allografts and in the graft-versus-host disease. The survival of renal allografts was prolonged in cynomolgus monkey recipients treated with the anti-Tac monoclonal antibody.

In patient studies, intravenously administered anti-Tac is added to conventional immunosuppression to prevent allograft rejection. The patients receive anti-Tac monoclonal antibody by intravenous adminstration in 100 ml of glucose or saline with 5% albumin carrier over about 2 hours. The patients are treated with about 20 mg of anti-Tac daily for about 10 days between the first and tenth day after their receipt of the organ allograft.

Eight patients receiving renal allografts have been treated with the above protocol of anti-Tac in addition to conventional immunsupression. None of these patients manifested toxicity due to the anti-Tac monoclonal antibody. Furthermore, none of them have rejected the transplanted kidney.

## Claims

1. Use of anti-Tac monoclonal antibody conjugated with yttrium-90 in the preparation of a medicament for the treatment of T-cell mediated disorder in humans.

2. Use according to Claim 1, wherein the T-cell mediated disorder is Adult T-cell Leukemia.

## Patentansprüche

1. Verwendung eines monoklonalen Anti-Tac-Antikörpers, der mit Yttrium-90 konjugiert ist, zur Herstellung eines Medikaments für die Behandlung von durch T-Zellen vermittelten Störungen beim Menschen.

2. Verwendung nach Anspruch 1, bei dem die durch T-Zellen vermittelte Störung eine T-Zellen-Leukämie des Erwachsenen ist.

## Revendications

1. Utilisation d'un anticorps monoclonal anti Tac conjugué avec de l'yttrium-90 pour la préparation d'un médicament destiné au traitement d'affections entraînées par les cellules T chez l'homme.

2. Utilisation selon la revendication 1, dans laquelle l'affection entraînée par les cellules T est une leucémie à cellules T de l'adulte.
